# EUROPEAN PATENT SPECIFICATION

(11) **EP 3 471 619 B1**
(45) Date of publication and mention of the grant of the patent: **05.08.2020**
(21) Application number: 17728866.9
(22) Date of filing: 12.06.2017
(51) Int. Cl.: A61B 8/00, A61B 8/08, G01S 15/89

(54) **IMAGE ORIENTATION IDENTIFICATION FOR AN EXTERNAL MICROCONVEX-LINEAR ULTRASOUND PROBE**
ERKENNUNG DER BILDAUSRICHTUNG FÜR EINE EXTERNE MIKROKONVEX-LINEARE ULTRASCHALLSONDE
IDENTIFICATION D'UNE ORIENTATION D'IMAGE DESTINÉE À UNE SONDE ULTRASONORE EXTERNE MICROCONVEXE-LINÉAIRE

(30) Priority: 16.06.2016 US 201662350848 P
(43) Date of publication of application: 24.04.2019
(73) Proprietor: Koninklijke Philips N.V., 5656 AG Eindhoven (NL)
(72) Inventor: FREEMAN, Steven, Russell, 5656 AE Eindhoven (NL)
(74) Representative: Philips Intellectual Property & Standards
(86) International application number: PCT/EP2017/064209
(87) International publication number: WO 2017/216078

(56) References cited:
- WO-A1-95/28129
- WO-A1-2015/099835
- WO-A2-98/38486
- US-A1- 2009 312 643

## Description

This invention relates to medical diagnostic ultrasonic systems and, in particular, to microconvex-linear ultrasound probes for biopsy procedures.

Ultrasonic image guidance is frequently used to guide biopsies and other needle procedures by which a needle is introduced into the body to biopsy or aspirate or ablate material inside the body. A familiar problem occurs at the beginning of the procedure, where it is desired to image the needle as soon as it penetrates the skin surface so that the path of the needle to the target can be guided and observed. It is desirable to be able to visualize and avoid penetrating superficial blood vessels and nerves to as great a degree as possible.
Furthermore, the presence of dense subcutaneous tissues can cause the needle to bend or deflect and vary from its intended path of travel. It is therefore desirable to begin imaging the needle as soon as it enters the body so that these potential problems can be immediately observed and overcome.

A common aid in handling the needle is to use a biopsy guide. This is a bracket that fastens around the ultrasound probe and holds the needle in-line for its intended path of travel. Unfortunately the biopsy guide worsens the problem of initial visualization of the needle, as it usually holds the needle outward from the side of the probe and away from the acoustic window of the probe. Other approaches have been tried to reduce this problem, such as manufacturing slots in the probe face next to the array transducer and sometimes even between elements of the array. However these approaches in specialized probe construction are expensive, such probes are difficult to clean, and are limited to the specific needle access of the particular design. Accordingly, many experienced clinicians prefer to use a standard imaging probe with no biopsy guide so that they can insert the needle into the skin of the patient as close to the probe and its acoustic window as possible. Accordingly it is desirable to facilitate both unaided and biopsy guide assisted biopsies with good image guidance.

It is a further objective of the present invention to improve visual guidance of needle insertion by providing a wide lateral field of view near the skin surface for needle insertion, and well resolved images at deeper depths of field as the needle approaches the target pathology inside the body.

Document US 2009/0312643 discloses an ultrasonic probe having a first and second group of ultrasonic transducers having different curvatures from each other.

WO 2015/099835 discloses a system and method for displaying ultrasound images having the orientation of the displayed anatomy change based on the orientation of the probe and/or display.

The invention is defined by the claims.

In accordance with the principles of the present invention, an external probe for image guidance of needle insertion procedures has a combined microconvex array and linear array construction. The probe has a handle by which a user can press the microconvex array section against the skin of the patient at the beginning of the procedure to visualize needle insertion, then can rotate the probe to bring the linear array section into good acoustic contact with the skin of the patient to observe the needle as it penetrates to deeper depths of the body. A method of using the probe in a needle procedure comprises pressing the microconvex array section against the subject to image with the microconvex array; inserting a needle adjacent to the microconvex array section and observing its initial penetration; rotating the probe to bring the linear array section into good acoustic contact with the subject; and observing deeper penetration of the needle with the linear array section.

In accordance with a further aspect of the present invention, an orientation processor circuit controls the display of the ultrasonic image so that the skin line of the patient is always located at the top of the display while the probe is rotated during needle insertion and depth penetration. The orientation processor may utilize an accelerometer producing signals which are processed to determine the direction of the force of gravity, image processing, or acoustic contact to determine the desired image orientation.

In the drawings:
FIGURE 1 illustrates needle insertion visually guided by a microconvex array probe.
FIGURE 2 illustrates needle insertion visually guided by a linear array probe.
FIGURE 3 illustrates the microconvex and linear acoustic apertures of an external ultrasound probe constructed in accordance with the principles of the present invention.
FIGURE 4 is a schematic illustration of the internal construction of an external ultrasound probe of the present invention.
FIGURE 5 is a flowchart of the steps of an invasive needle procedure conducted in accordance with the present invention.
FIGURE 6 illustrates initial needle insertion guidance using a microconvex-linear array probe of the present invention.
FIGURE 7 illustrates guidance of deeper needle insertion using a microconvex-linear array probe of the present invention.
FIGURES 8a and 8b illustrate the change in image orientation which occurs as a microconvex-linear array probe of the present invention is rotated to follow needle insertion.
FIGURES 9a, 9b, 9c, and 9d illustrate algorithms executed by an orientation processor in accordance with the present invention to determine image orientation.
FIGURE 10 illustrates an ultrasound system in block diagram with an image orientation processor in accordance with the present invention.

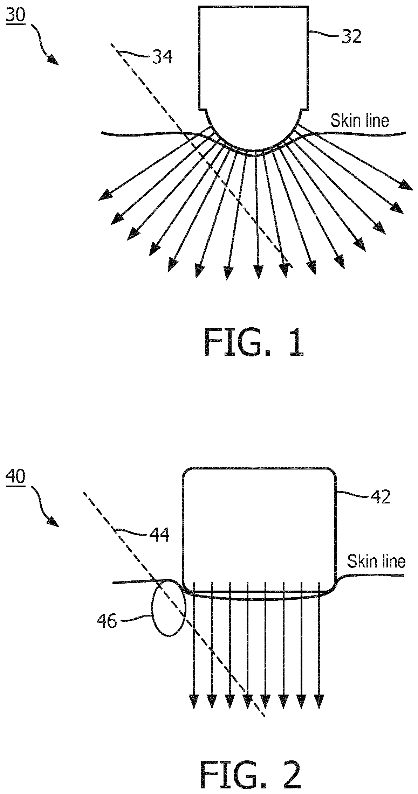

FIGURE 1 illustrates a needle insertion procedure with visual guidance provided by a microconvex transducer array probe 30 having a microconvex array 32 at its distal tip. The term "microconvex" is applied to curved array transducers that are tightly curved with a relatively small radius of curvature. Microconvex arrays are generally used in delicate procedures when a small probe with a wide field of view is needed. Microconvex ultrasound transducers provide a wide field of view immediately beneath the skin line and thus are desirable for needle interventions. The procedure is performed by pressing the microconvex array aperture of the probe 30 against the skin surface as shown in the drawing, and inserting a needle adjacent to the probe and, for a two-dimensional imaging probe, in line with the plane of the image. The needle is inserted into the body at an angle as indicated by needle path 34. The arrows inside the body indicate the beam directions from the microconvex array 32. Although the needle can be followed visually almost immediately after penetrating the skin line, the limited active aperture of the microconvex array due to element directivity and the array curvature limits the resolution of the image at depth. Thus, linear array probes are frequently used for needle guidance because they provide better penetration and resolution at deeper depth than microconvex probes. FIGURE 2 illustrates a linear array transducer probe 40 with a linear array transducer 42 at its distal end. A linear array is generally able to visually follow the path 44 of the needle to a considerable depth in the body, as indicated by the arrows extending from the array aperture into the body. But with a standard linear array probe, some of the needle path 46 at the initial point of entry adjacent to the probe 40 is not visualized at all and the beams of a linear array produce poorer resolution at the edge of the array.

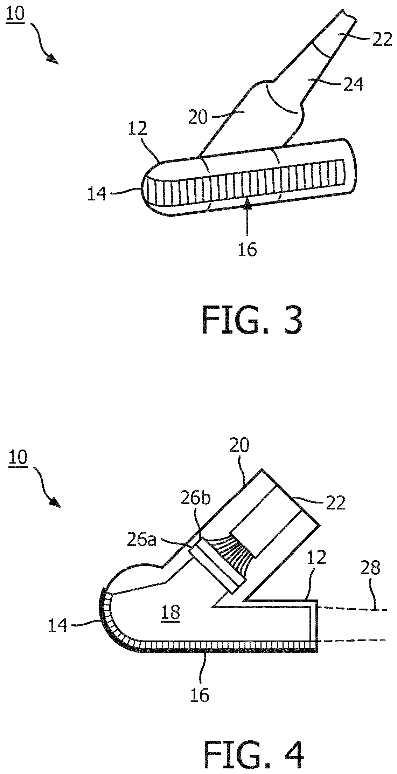

FIGURE 3 illustrates a microconvex-linear array transducer probe 10 constructed in accordance with the principles of the present invention. The probe 10 has a main body 12 with an active aperture of transducer elements extending from a straight edge of the main body, down to and around a distal tip of the main body. The transducer elements thus comprise a linear array 16 where the section of elements is in a straight line, transitioning to a microconvex array 14 where the elements curve around the distal tip of the probe. With beamforming that transmits and receives beams normal to the active aperture surface all along the array of elements, a continuous image field can be scanned and imaged in front of the entire section of microconvex and linear elements. On the opposite side of the main body 12 from the linear array section is a handle 20 which extends from the main body at an oblique angle and is used to hold the probe 10 in contact with the skin surface during a needle procedure. In this example a cable 22 which connects the probe 10 to an ultrasound imaging system exits the probe through the end of the handle 20. The cable is protected at its point of attachment to the handle with a cable strain relief 24.

The internal components of the probe 10 are shown in the cross sectional view of FIGURE 4. In this view the microconvex elements 14 curve around the distal tip of the probe on the left side and transition into a linear array of elements 16. Attached to the back of the array is a flex circuit 18 with conductors attached to the array elements. The conductors of the flex circuit terminate at a connector 26a inside the handle portion 20 of the probe. The cable 22 entering the end of the handle has conductors terminating in a connector 26b, which mates with connector 26a to electrically couple the array elements to the conductors of the cable and ultimately to the beamformer of the ultrasound system. While the cable 22 is shown attached at the end of the handle 20 in this example, it could alternately be attached to the probe at the proximal end of the main body 12 as indicated by the dashed lines 28 in the drawing.

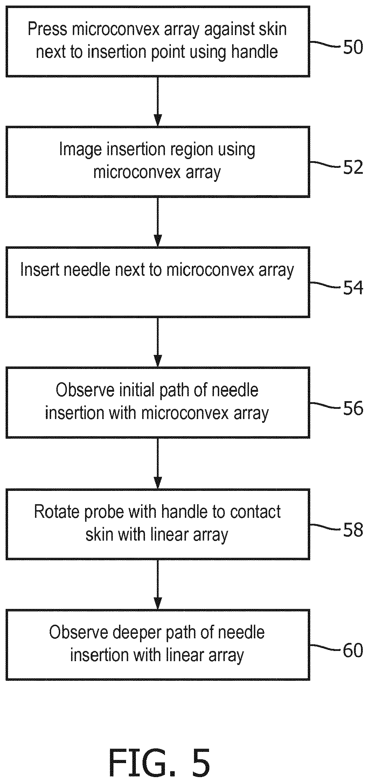

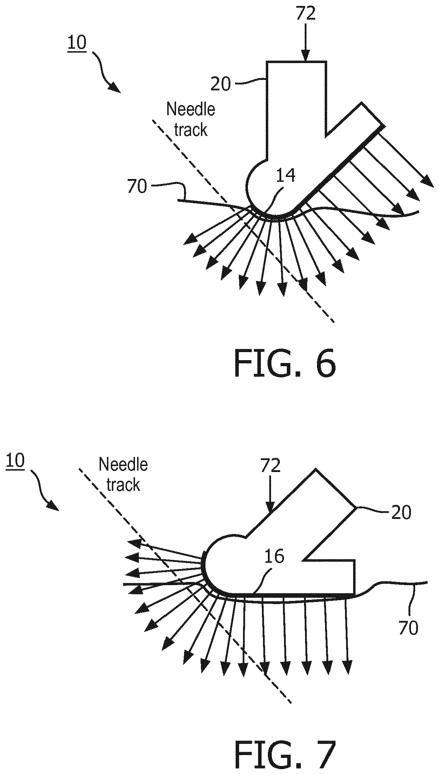

FIGURE 5 is a flowchart illustrating the steps in a typical needle insertion procedure in accordance with the present invention. In the first step 50 a clinician grasps the handle 20 of the probe and presses the microconvex array 14 into good acoustic contact with the skin of the patient. When the probe is held in this way it appears as shown in FIGURE 6. In this position the clinician is able to assert contact force in the direction of the axis of the handle and directly in line with the microconvex array 14 as shown at 72 in the drawing. The force of the probe against the skin 70 of the patient will not only assure good acoustic contact between the microconvex array and the skin, it also will widen the contact area due to depression of the skin. This enables scanning in a wider sector by reason of the improved contact at the edge of the probe, a sector which is able to image the initial penetration of the needle into the body as indicated by the dashed needle track shown in the drawing, which is step 52 in FIGURE 5. In step 54 the clinician inserts the needle next to the microconvex array 14 as shown in FIGURE 6, and in step 56 the clinician observes the initial path of needle insertion in the image field scanned by the microconvex array. As the clinician advances the needle, the next step 58 is to rotate the probe with the handle 20, bringing the linear array 16 into acoustic contact with the skin 70 as shown in FIGURE 7. This rotation also is seen to bring the far end of the microconvex array out of acoustic contact with the skin. This may be done without losing the view of the needle, as at least a portion of the aperture of the microconvex and linear array elements is always in acoustic contact with the skin as the probe is rotated. The handle 20 is now above the linear array 16 as FIGURE 7 shows, enabling the clinician to press down with a force 72 to firmly press the linear array aperture into good acoustic contact with the skin 70. The continued insertion of the needle is beneath the linear array section of the probe aperture, enabling the linear array 16 to visualize continued insertion of the needle deeper into the body with good resolution and clarity until the tip of the needle reaches its intended target, as stated by step 60.

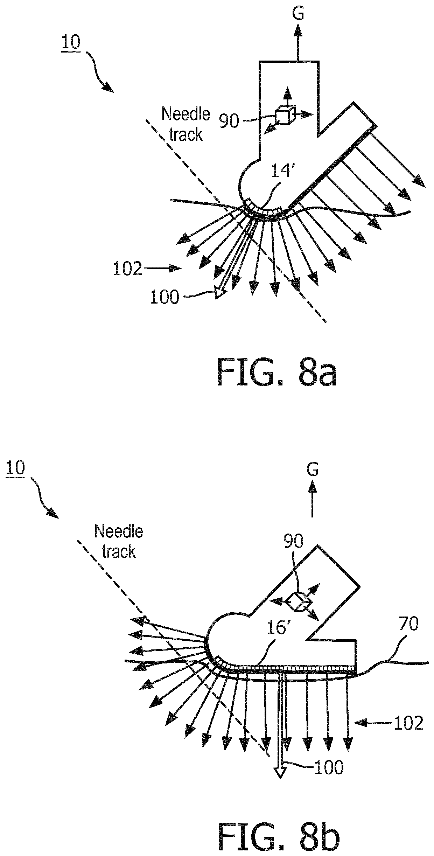

Due to the fact that the probe is intended to be rotated during the needle insertion procedure, and also the fact that the microconvex-linear array transmits and receives beams in directions spanning over 100°, an ambiguity arises during imaging: how should the image be displayed? Ultrasound images are normally displayed in a fixed orientation to the probe, with the shallowest beam depths at the top of the image and extending to deeper beam depths at the bottom. But when the probe is rotated during the procedure as illustrated in FIGURES 6 and 7, the display becomes disorienting as the needle position appears to move on the screen. Since the clinician is intently focused on the needle position to guide the needle tip to its target tissue in the body, it is desirable to prevent this disorientation. In accordance with a further aspect of the present invention, the display format is dynamically adjusted during rotation of the probe so that greater tissue depths are always at the bottom of the display, thereby giving the clinician a consistent frame of reference. Preferably this is done by control of the manner in which the scan converter renders the image. In a conventional ultrasound system the purpose of the scan converter is to convert the r-θ coordinates of the receive beam scanlines into an image with x-y coordinates suitable for a raster display and in the appropriate sector, linear, or curved linear scan format. In an implementation of the present invention, the scan converter is further controlled by an orientation signal which identifies the vertical orientation (up, down direction) of the image. There are several ways to accomplish this. One is by use of an accelerometer 90 located in the probe 10 as shown in FIGURES 8a and 8b, which produces signals that measure a constant orientation direction such as the direction of gravitational force. The scan converter then renders the ultrasound image with its vertical direction aligned with the measured orientation direction. Conventionally accelerometers have been used in ultrasound probes to measure displacement for the reconstruction of 3D images, as described in US Pat. 5,529,070 (Augustine et al.) In the probe described in this patent, signals from accelerometers are processed over time to measure probe displacement, which is the second derivative of the acceleration signals. For this dynamic acceleration measurement, the gravitational force vectors are canceled in the processing algorithms. But it is the static gravitational force vector alone which can be used as an orientation signal in an implementation of the present invention.

A second way to produce an orientation signal is by detection of the portion of the array 14, 16 which is acoustically coupled to the skinline at any point in time. The vertical image direction is then taken as a vector 100 normal to the center of the acoustically coupled portion of the array. For instance, FIGURE 8a shows the probe 10 being held at the time of needle insertion with most of the elements 14' of the microconvex array in contact with the skin. The elements of the linear array 16 are not in contact with the skinline at this time. A vector arrow 100 is shown drawn normal to the center of the microconvex array portion 14', and this vector direction is used for the image orientation signal. At a later point in the procedure, when the linear array portion 16' of the probe has been rotated into acoustic contact with the skinline 70, the vector arrow 100 normal to the center of the acoustically coupled portion 16' of the array appears as shown in FIGURE 8b. Scanlines 102 which are parallel to the vector arrow direction will be rendered vertically in the scan converted image, with scanlines at other angles oriented in accordance with their angular offset from the direction of the vector arrow direction of the orientation signal.

A third way to produce a suitable orientation signal is by image analysis, also known in the art as feature tracking. For instance the layers of skin, fat, and striated muscle immediately beneath the skin can be identified in the image and the horizontal orientation set to be in general alignment with these layers by an orientation signal. A second image analysis technique is to identify the pathological target of the needle procedure in the image, which may be done either manually or automatically. For example, the clinician can click on the target in the image prior to commencing needle insertion. The target anatomy is then rendered in the same location in each successive image frame, which may be done using image stabilization techniques. Ultrasonic image stabilization is well known, as described in US Pat. 6,589,176 (Jago et al.) The image stabilization in an implementation of the present invention is preferably not done rigorously enough to preclude rotation, as that is the expected result of the probe motion. Center-to-center stabilization will be sufficient to produce a sequence of consistently useful images as the probe is rotated. Alternatively, the speckle characteristic of the identified target anatomy can be tracked from frame to frame to maintain the anatomy in the same location from frame to frame.

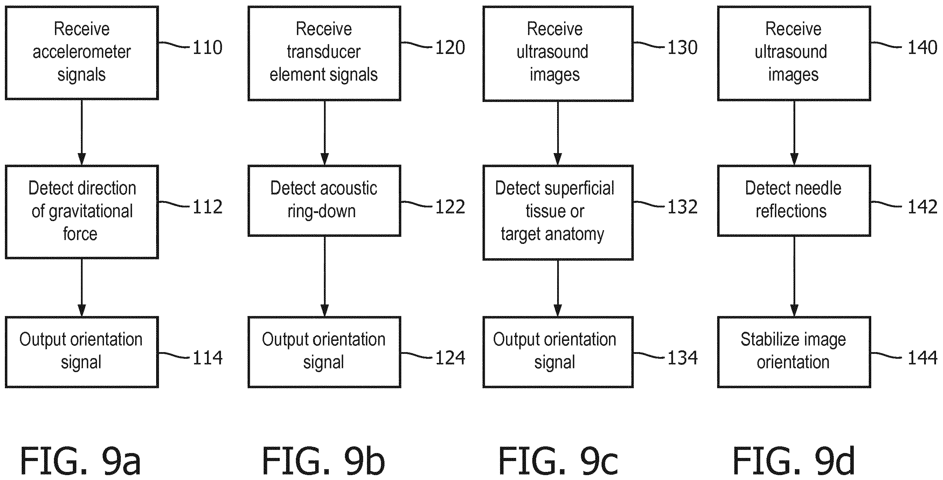

FIGURES 9a, 9b, 9c, and 9d illustrate a number of methods for generating an orientation signal. FIGURE 9a illustrates a technique using an accelerometer in the probe. A suitable accelerometer for this purpose is a three-axis accelerometer such as those of the ADXL300 series of MEMS (micro electro-mechanical system) accelerometers available from Analog Devices, Inc. of Boston, MA. The signals of the three axes are received by an orientation processor in step 110. Samples of these three signals are respectively averaged over a sampling interval such as a few seconds to produce values vₓ, v_{y}, and v_{z} from the three axes. The three values are vectorially combined to produce the vertical acceleration vector v corresponding to gravity, which is nominally 9.81 meters/second and is in a direction straight up as shown in step 112. The vector direction of gravitational force, indicated by arrow G in FIGURES 8a and 8b, is then used in the output orientation signal, step 114, to cause the scan converter to render the image with the indicated direction G as the vertical direction. Thus, the direction commonly referred to as "up" will always be at the top of the displayed image.

FIGURE 9b illustrates an orientation signal identification process using acoustic contact between elements of the transducer array 14, 16 and the skin of the patient. Signals from all of the elements of the array are coupled to an orientation processor in step 120 where they are analyzed for acoustic ring-down in step 122. When an ultrasound transducer element is not acoustically coupled to the skin, its echo response exhibits a distinctive ring-down artifact. A typical ring-down signal from an uncoupled transducer element is shown in Fig. 6 of US Pat. 5,517,994 (Burke et al.) for instance. Elements acoustically coupled to the subject will in contradistinction receive a sequence of echo signals from tissue. The orientation processor algorithm identifies those elements which are acoustically coupled to the patient, identifies the center of the sequence of acoustically coupled elements and, from knowledge of the geometry of the array, then identifies the direction normal to this center. The orientation signal communicates this direction (arrow 100 in FIGURES 8a and 8b) to the scan converter in step 124, which uses this direction as the vertical direction in the rendered images. The element coupling and arrow direction are constantly updated so that the vertical direction of the image is constantly refined during the needle insertion procedure.

FIGURE 9c illustrates the production of an image orientation signal by image processing and feature tracking. In step 130 a sequence of ultrasound images is received by the orientation processor which in this instance is an image processor. Analysis is performed in step 132 to locate known image features, such as the previously described superficial layers immediately beneath the skin or the target anatomy. Alternatively these image characteristics may be manually identified in an image. An identified characteristic is tracked and its orientation or image location is communicated to the scan converter in step 134, which renders the image characteristic consistently from image frame to image frame.

A specific image analysis technique is illustrated in FIGURE 9d. During needle insertion the clinician will be closely watching the position of the needle as it enters the body and particularly its inclination toward the target anatomy. The method of FIGURE 9d assists the clinician in this effort by stabilizing the position of the needle in the images. Ultrasound images are received by an orientation processor at step 140, which detects echo signal reflections from a needle. Such echo signals are very distinctive as a needle is a highly specular reflector of ultrasound and the echo signals from a needle are very strong. See, e.g., US Pat. 6,951,542 (Greppi et al.) When these distinctive echoes are detected by the orientation processor in step 142, their image locations are communicated to the scan converter in the orientation signal, which responds by rendering the needle in a consistent position from frame to frame. Image stabilization techniques can be used to render a sequence of images with a stable needle location. See, e.g., US Pat. 6,589,176 (Jago et al.)

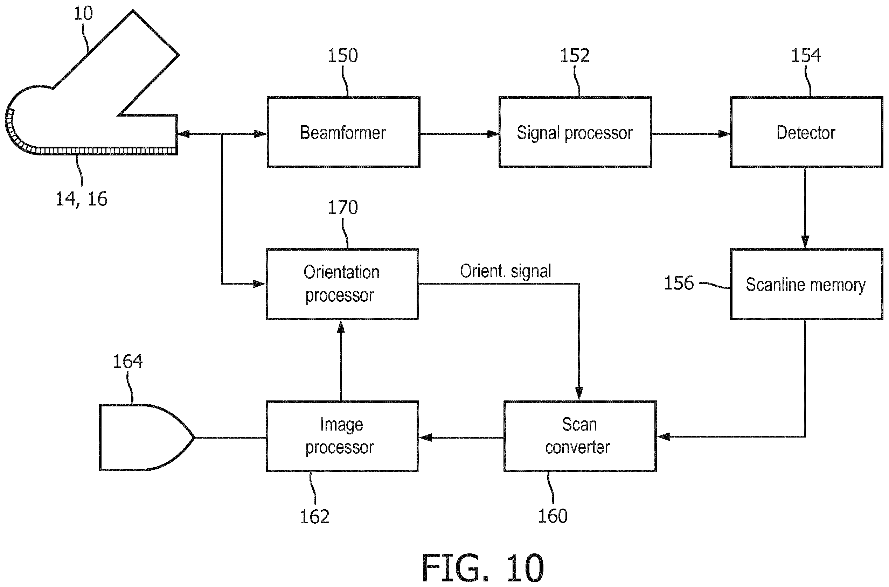

An ultrasound system constructed in accordance with the principles of the present invention is shown in block diagram form in FIGURE 10. The microconvex-linear array 14, 16 of a probe 10 is coupled to a beamformer 150, which causes elements of the array to transmit ultrasound waves and receive echo signals in response. The received echo signal are beamformed into scanlines of coherent echo signals by the beamformer. The echo signals are processed by a signal processor 152 which performs functions such as filtering, frequency or spatial compounding, harmonic separation, and quadrature demodulation. A detector 154 performs signal detection, amplitude detection in the case of B mode images and Doppler detection in the case of Doppler signals. The scanlines of echo signals are stored in a scanline memory 156 which may be a conventional digital memory device. The scanlines of echo signals are rendered in a desired image format of Cartesian coordinates by a scan converter 160, with the vertical axis of the image or the location of a specific image object determined by an orientation signal as described previously. The orientation signal is produced as described above by an orientation processor 170, which may comprise electronic hardware components, hardware controlled by software, or a microprocessor executing signal and/or image processing algorithms as described in conjunction with FIGURES 9a-9d. The orientation processor 170 is shown coupled to receive accelerometer signals and/or echo signals from the probe 10 for vertical vector analysis and/or acoustic coupling analysis as described in FIGURES 9a and 9b. The orientation processor 170 is also shown coupled to receive ultrasound images from an image processor 162 for execution of the image processing techniques for orientation signal production as described in conjunction with FIGURES 9c and 9d. The image processor 162 receives rendered ultrasound images from the scan converter 160 and applies the images to a monitor or display 164 for viewing by the clinician.

It should be noted that the various embodiments described above and illustrated herein may be implemented in hardware, software or a combination thereof. The various embodiments and/or components, for example, the modules, or components and controllers therein, also may be implemented as part of one or more computers or microprocessors. The computer or processor may include a computing device, an input device, a display unit and an interface, for example, for accessing the Internet. The computer or processor may include a microprocessor. The microprocessor may be connected to a communication bus, for example, to access a PACS system. The computer or processor may also include a memory. The memory may include Random Access Memory (RAM) and Read Only Memory (ROM). The computer or processor further may include a storage device, which may be a hard disk drive or a removable storage drive such as a floppy disk drive, optical disk drive, solid-state thumb drive, and the like. The storage device may also be other similar means for loading computer programs or other instructions into the computer or processor.

As used herein, the term "computer" or "module" or "processor" may include any processor-based or microprocessor-based system including systems using microcontrollers, reduced instruction set computers (RISC), ASICs, logic circuits, and any other circuit or processor capable of executing the functions described herein. The above examples are exemplary only, and are thus not intended to limit in any way the definition and/or meaning of these terms. The computer or processor executes a set of instructions that are stored in one or more storage elements, in order to process input data. The storage elements may also store data or other information as desired or needed. The storage element may be in the form of an information source or a physical memory element within a processing machine.

The set of instructions may include various commands that instruct the computer or processor as a processing machine to perform specific operations such as the methods and processes of the various embodiments of the invention. The set of instructions may be in the form of a software program. The software may be in various forms such as system software or application software and which may be embodied as a tangible and non-transitory computer readable medium. Further, the software may be in the form of a collection of separate programs or modules, a program module within a larger program or a portion of a program module. The software also may include modular programming in the form of object-oriented programming. The processing of input data by the processing machine may be in response to operator commands, or in response to results of previous processing, or in response to a request made by another processing machine.

Furthermore, the limitations of the following claims are not written in means-plus-function format and are not intended to be interpreted based on 35 U.S.C. 112, sixth paragraph, unless and until such claim limitations expressly use the phrase "means for" followed by a statement of function devoid of further structure.

## Claims

1. An ultrasound system comprising:
a microconvex-linear ultrasound probe (10) comprising an array of microconvex ultrasound elements (14) and linear ultrasound elements (16);
a scan converter (160), coupled to receive echo signals from the ultrasound probe and render ultrasound images in a desired image format, wherein the echo signals form beams normal to active aperture surfaces along the entire array of microconvex elements and linear elements such that a continuous image field is scanned along the entire array of microconvex elements and linear elements;
an orientation processor (170), coupled to at least one of the ultrasound probe or the scan converter to produce an image orientation signal which is coupled to the scan converter; and
an ultrasound image display (164) coupled to display images produced by the scan converter in a desired image orientation.

2. The ultrasound system of Claim 1, wherein the ultrasound probe (10) further comprises an accelerometer (90),
wherein the orientation processor (170) is further coupled to receive signals from the accelerometer.

3. The ultrasound system of Claim 2, wherein the orientation processor (170) is further configured to detect the direction of gravitational force.

4. The ultrasound system of Claim 1, wherein the ultrasound probe (10) comprises an array of microconvex-linear transducer elements,
wherein the orientation processor (170) is further coupled to receive echo signals from the transducer elements.

5. The ultrasound system of Claim 4, wherein the orientation processor (170) is further configured to identify ring-down signals from elements which are not acoustically coupled to a subject.

6. The ultrasound system of Claim 1, wherein the orientation processor (170) further comprises an ultrasound image processor (162).

7. The ultrasound system of Claim 6, wherein the orientation processor (170) is further configured to identify a specific feature in an ultrasound image.

8. The ultrasound system of Claim 7, wherein the orientation processor (170) is further configured to track a specific feature in a sequence of ultrasound images.

9. The ultrasound system of Claim 8, wherein the orientation processor (170) is further configured to track the specific feature in a sequence of ultrasound images by speckle tracking.

10. The ultrasound system of Claim 8, wherein the orientation processor (170) is further configured to stabilize the location of the specific feature in the sequence of ultrasound images.

11. The ultrasound system of Claim 7, wherein the orientation processor (170) is further configured to identify echo signals returned from a needle.

12. The ultrasound system of Claim 11, wherein the orientation processor (170) is further configured to stabilize the location of the needle in a sequence of ultrasound images.

13. The ultrasound system of Claim 1, further comprising a beamformer (150) coupled to receive echo signals from transducer element of an array of transducer elements in the microconvex-linear ultrasound probe (10).

14. The ultrasound system of Claim 13, further comprising a detector (154) coupled to the beamformer.

15. The ultrasound system of Claim 14, further comprising a scanline memory (156) coupled to the detector.

## Patentansprüche

1. Ein Ultraschallsystem, das Folgendes umfasst:
eine mikrokonvex-lineare Ultraschallsonde (10) mit einer Anordnung von mikrokonvexen Ultraschallelementen (14) und linearen Ultraschallelementen (16);
einen Scan-Konverter (160), der angeschlossen ist, um Echosignale von der Ultraschallsonde zu erhalten und Ultraschallbilder im gewünschten Bildformat darzustellen, wobei die Echosignale für die aktiven Apertur-Oberflächen entlang der gesamten Anordnung von mikrokonvexen und linearen Elementen gewöhnliche Strahlen bilden, sodass ein fortlaufendes Bildfeld entlang der gesamten Anordnung von mikrokonvexen und linearen Elementen abgetastet wird;
einen Ausrichtungsprozessor (170), der mindestens entweder an die Ultraschallsonde oder den Scan-Konverter angeschlossen ist, um ein Bildausrichtungssignal zu erzeugen, das mit dem Scan-Konverter verbunden ist; und
eine Ultraschallbildanzeige (164), die angeschlossen ist, um die vom Scan-Konverter erstellten Bilder in der gewünschten Bildausrichtung anzuzeigen.

2. Das Ultraschallsystem gemäß Anspruch 1, wobei die Ultraschallsonde (10) zudem einen Beschleunigungsmesser (90) umfasst, wobei der Ausrichtungsprozessor (170) zudem angeschlossen ist, um Signale vom Beschleunigungsmesser zu empfangen.

3. Das Ultraschallsystem gemäß Anspruch 2, wobei der Ausrichtungsprozessor (170) zudem die Ausrichtung der Schwerkraft erkennt.

4. Das Ultraschallsystem gemäß Anspruch 1, wobei die Ultraschallsonde (10) eine Anordnung von mikrokonvex-linearen Wandlern umfasst, wobei der Ausrichtungsprozessor (170) zudem angeschlossen ist, um Echosignale von den Wandlern zu empfangen.

5. Das Ultraschallsystem gemäß Anspruch 4, wobei der Ausrichtungsprozessor (170) zudem Ring-Down-Signale von Elementen erkennt, die nicht akustisch an einem Objekt angeschlossen sind.

6. Das Ultraschallsystem gemäß Anspruch 1, wobei der Ausrichtungsprozessor (170) zudem einen Ultraschallbildprozessor (162) umfasst.

7. Das Ultraschallsystem gemäß Anspruch 6, wobei der Ausrichtungsprozessor (170) zudem ein bestimmtes Merkmal in einem Ultraschallbild erkennt.

8. Das Ultraschallsystem gemäß Anspruch 7, wobei der Ausrichtungsprozessor (170) zudem ein bestimmtes Merkmal in einer Abfolge von Ultraschallbildern nachverfolgt.

9. Das Ultraschallsystem gemäß Anspruch 8, wobei der Ausrichtungsprozessor (170) zudem das bestimmte Merkmal in einer Abfolge von Ultraschallbildern mithilfe von Speckle-Tracking nachverfolgt.

10. Das Ultraschallsystem gemäß Anspruch 8, wobei der Ausrichtungsprozessor (170) zudem die Position des bestimmten Merkmals in der Abfolge von Ultraschallbildern stabilisiert.

11. Das Ultraschallsystem gemäß Anspruch 7, wobei der Ausrichtungsprozessor (170) zudem Echosignale erkennt, die von einer Nadel zurückgegeben werden.

12. Das Ultraschallsystem gemäß Anspruch 11, wobei der Ausrichtungsprozessor (170) zudem die Position der Nadel in einer Abfolge von Ultraschallbildern stabilisiert.

13. Das Ultraschallsystem gemäß Anspruch 1, das zudem über einen Beamformer (150) verfügt, der angeschlossen ist, um Echosignale von einem Wandler einer Anordnung von Wandlern in der mikrokonvex-linearen Ultraschallsonde (10) zu empfangen.

14. Das Ultraschallsystem gemäß Anspruch 13, das zudem einen am Beamformer angeschlossenen Detektor (154) umfasst.

15. Das Ultraschallsystem gemäß Anspruch 14, das zudem einen am Detektor angeschlossenen Scanline-Speicher (156) umfasst.

## Revendications

1. Système à ultrasons comprenant :
une sonde ultrasonore microconvexe linéaire (10) comprenant un réseau d'éléments ultrasonores microconvexe (14) et d'éléments ultrasonores linéaires (16) ;
un convertisseur de balayage (160), couplé pour recevoir des signaux d'écho de la sonde ultrasonore et pour rendre des images par ultrasons dans un format d'image souhaité, dans lequel les signaux d'écho forment des faisceaux de surfaces d'ouverture normales à actives le long de l'ensemble du réseau d'éléments microconvexe et d'éléments linéaires de telle sorte qu'un champ d'image continu est balayé le long de l'ensemble du réseau d'éléments microconvexe et d'éléments linéaires ;
un processeur d'orientation (170), couplé à la sonde ultrasonore et/ou le convertisseur de balayage pour produire un signal d'orientation d'image, lequel est couplé au convertisseur de balayage ; et
un afficheur d'image par ultrasons (164) couplé pour afficher des images produites par le convertisseur de balayage dans une orientation d'image souhaitée.

2. Système à ultrasons selon la revendication 1, dans lequel la sonde ultrasonore (10) comprend en outre un accéléromètre (90), dans lequel le processeur d'orientation (170) est en outre couplé pour recevoir des signaux de l'accéléromètre.

3. Système à ultrasons selon la revendication 2, dans lequel le processeur d'orientation (170) est en outre configuré pour détecter la direction de la force gravitationnelle.

4. Système à ultrasons selon la revendication 1, dans lequel la sonde ultrasonore (10) comprend un réseau d'éléments transducteurs microconvexe linéaires, dans lequel le processeur d'orientation (170) est en outre couplé pour recevoir des signaux d'écho des éléments transducteurs.

5. Système à ultrasons selon la revendication 4, dans lequel le processeur d'orientation (170) est en outre configuré pour identifier les signaux de sonnerie descendante des éléments, lesquels ne sont pas couplés de manière acoustique à un sujet.

6. Système à ultrasons selon la revendication 1, dans lequel le processeur d'orientation (170) comprend en outre un processeur d'image par ultrasons (162) .

7. Système à ultrasons selon la revendication 6, dans lequel le processeur d'orientation (170) est en outre configuré pour identifier une caractéristique spécifique dans une image par ultrasons.

8. Système à ultrasons selon la revendication 7, dans lequel le processeur d'orientation (170) est en outre configuré pour suivre une caractéristique spécifique dans une séquence d'images par ultrasons.

9. Système à ultrasons selon la revendication 8, dans lequel le processeur d'orientation (170) est en outre configuré pour suivre la caractéristique spécifique dans une séquence d'images par ultrasons par suivi de granularité.

10. Système à ultrasons selon la revendication 8, dans lequel le processeur d'orientation (170) est en outre configuré pour stabiliser l'emplacement de la caractéristique spécifique dans la séquence d'images par ultrasons.

11. Système à ultrasons selon la revendication 7, dans lequel le processeur d'orientation (170) est en outre configuré pour identifier les signaux d'écho renvoyés par une aiguille.

12. Système à ultrasons selon la revendication 11, dans lequel le processeur d'orientation (170) est en outre configuré pour stabiliser l'emplacement de l'aiguille dans une séquence d'images par ultrasons.

13. Système à ultrasons selon la revendication 1, comprenant en outre un formateur de faisceau (150) couplé pour recevoir des signaux d'écho provenant d'un élément transducteur d'un réseau d'éléments transducteurs dans la sonde ultrasonore microconvexe linéaire (10).

14. Système à ultrasons selon la revendication 13, comprenant en outre un détecteur (154) couplé au formateur de faisceau.

15. Système à ultrasons selon la revendication 14, comprenant en outre une mémoire de ligne de balayage (156) couplée au détecteur.
